(19) Europäisches Patentamt
European Patent Office
Office européen des brevets

(11) Veröffentlichungsnummer: **0 282 865 B1**

(12) EUROPÄISCHE PATENTSCHRIFT

(45) Veröffentlichungstag der Patentschrift: **02.01.92**

(51) Int. Cl.[5]: **C07D 249/12**, C07D 285/12, C07D 213/70, G03C 1/30

(21) Anmeldenummer: **88103514.1**

(22) Anmeldetag: **07.03.88**

Die Akte enthält technische Angaben, die nach dem Eingang der Anmeldung eingereicht wurden und die nicht in dieser Patentschrift enthalten sind.

# (54) Härtungsmittel für Proteine, eine damit gehärtete Bindemittelschicht und ein eine solche Schicht enthaltendes fotografisches Aufzeichnungsmaterial.

(30) Priorität: **17.03.87 DE 3708541**

(43) Veröffentlichungstag der Anmeldung: **21.09.88 Patentblatt 88/38**

(45) Bekanntmachung des Hinweises auf die Patenterteilung: **02.01.92 Patentblatt 92/01**

(84) Benannte Vertragsstaaten: **BE DE FR GB IT**

(56) Entgegenhaltungen:
**EP-A- 0 031 959**
**EP-A- 0 098 454**
**EP-A- 0 207 399**

(73) Patentinhaber: **Agfa-Gevaert AG**
**W-5090 Leverkusen 1(DE)**

(72) Erfinder: **Himmelmann, Wolfgang, Dr.**
**Im Ziegelfeld 7**
**W-5090 Leverkusen 3(DE)**
Erfinder: **Sobel, Johannes, Dr.**
**Willi-Baumeister-Strasse 9**
**W-5090 Leverkusen 1(DE)**
Erfinder: **Öhlschläger, Hans, Dr.**
**Am Katterbach 34**
**W-5060 Bergisch-Gladbach 2(DE)**
Erfinder: **Schranz, Karl-Wilhelm, Dr.**
**Schillerstrasse 1**
**W-5068 Odenthal-Hahnenberg(DE)**

EP 0 282 865 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft sulfonylethylsulfatgruppenhaltige heteroaromatische Verbindungen mit oder ohne Vinylsulfongruppen als Härtungsmittel für Proteine, gehärtete Bindemittelschichten, deren Bindemittel ein Protein enthält und insbesondere ein lichtempfindliches fotografisches Aufzeichnungsmaterial, dessen proteinhaltige Bindemittelschichten mit dem erfindungsgemäßen Härtungsmittel gehärtet sind.

Als Härtungsmittel für Proteine und im besonderen für Gelatine sind bereits zahlreiche Substanzen beschrieben worden. Hierzu gehören beispielsweise Metallsalze wie Chrom-, Aluminium- oder Zirkonsalze, Aldehyde und halogenhaltige Aldehydverbindungen, insbesondere Formaldehyd, Dialdehyde und Mucochlorsäure, 1,2- und 1,4-Di-ketone wie Cyclohexandion-1,2 und Chinone sowie Chloride oder Anhydride von mehrbasigen organischen Säuren, wie Anhydride von Tetracarbonsäuren, Verbindungen mit mehreren reaktionsfähigen Vinylgruppen wie Vinylsulfone, Acrylamide, Verbindungen mit mindestens zwei leicht spaltbaren, heterocyclischen 3-gliedrigen Ringen wie Ethylenoxid und Ethylenimin, mehrfunktionelle Methansulfonsäureester und Bis-$\alpha$-chloracylamidoverbindungen.

In neuerer Zeit wurden hochmolekulare Härtungsmittel, wie Polyacrolein bzw. seine Derivate oder Mischpolymerisate sowie Alginsäurederivate bekannt, die speziell als schichtbegrenzte Härtungsmittel Verwendung finden.

Die Verwendung der genannten Verbindungen für fotografische Zwecke ist jedoch mit einer Reihe schwerwiegender Nachteile verbunden. Einige dieser Verbindungen sind fotografisch aktiv und deshalb zur Härtung fotografischer Aufzeichnungsmaterialien ungeeignet, andere beeinflussen die physikalischen Eigenschaften, z.B. die Brüchigkeit der Gelatineschichten so nachteilig, daß sie nicht verwendet werden können. Andere wiederum verursachen Färbungen oder eine Änderung des pH-Wertes während der Härtungsreaktion. Darüber hinaus ist es für die Härtung fotografischer Schichten besonders wichtig, daß die Härtung möglichst kurze Zeit nach dem Auftrocknen ihr Maximum erreicht, damit sich nicht, wie im Falle der Mucochlorsäure oder des Formaldehyds, die Durchlässigkeit des zu härtenden Materials für die Entwicklerlösung fortlaufend ändert.

In gewissen Fällen haben Vernetzungsmittel für Gelatine auch hautschädigende Wirkung, wie Ethyleniminverbindungen, so daß ihre Anwendung schon aus physiologischen Gründen nicht angebracht ist.

Es ist weiter bekannt, Trichlortriazin, Hydroxydichlortriazin und Dichloraminotriazine als Härtungsmittel zu verwenden. Nachteilig sind hierbei der verhältnismäßig hohe Dampfdruck, die Freisetzung von Salzsäure während der Härtung und die physiologische Wirkung dieser Verbindungen. Wasserlösliche Derivate, die Carboxyl- und Sulfonsäuregruppen enthalten und die durch Umsetzung von Cyanurchlorid mit einem Mol Diaminoalkyl- oder Diaminoarylsulfonsäure oder Carbonsäure erhalten werden, zeigen diese Nachteile nicht und sind deshalb in neuerer Zeit als Härtungsmittel vorgeschlagen worden. Ihre praktische Verwendbarkeit ist jedoch begrenzt, da sie sich infolge ihrer guten Löslichkeit beim Stehen in wäßrigen Lösungen zersetzen und dadurch schnell an Wirksamkeit verlieren.

Es ist schließlich bei einem Härtungsmittel für fotografische gelatinehaltige Schichten sowohl aus Herstellungs- als auch aus Verarbeitungsgründen von größter Bedeutung, daß das Einsetzen der Vernetzungsreaktion in gewissen Grenzen bestimmbar ist, beispielsweise durch Wahl der Trocknungstemperatur oder des pH-Wertes.

Als Härtungsmittel für fotografische Gelatinschichten sind auch Verbindungen mit 2 oder mehreren Acrylamidgruppen im Molekül bekannt, z.B. N,N′,N″-Trisacryloylhexahydrotriazin oder Methylenbisacrylamid.

Die Härtungswirkung dieser Verbindungen ist nach einiger Zeit zwar gut, jedoch sind die Verbindungen in Wasser schwer löslich, was innerhalb der Schicht zu Ungleichmäßigkeiten in der Härtung führen kann.

Besondere Probleme ergeben sich bei der in zunehmendem Maße gebrauchten Schnellverarbeitung fotografischer, insbesondere farbfotografischer Aufzeichnungsmaterialien, die gesteigerte Anforderungen an die mechanischen Eigenschaften und das Quellverhalten der Aufzeichnungsmaterialien stellt. Dazu kommen die Schwierigkeiten, die sich aus der Notwendigkeit ergeben, immer dünnere fotografische Schichten herzustellen. Man hat versucht, solche Probleme durch Anwendung verschiedenartiger Härtungsmittel zu lösen. Die bekannten Härtungsmittel haben dabei entweder neue Schwierigkeiten verursacht oder sich einfach als ungeeignet erwiesen. Zu diesen Härtungsmitteln zählen die zahlreichen bekannten Vinylsulfongruppen enthaltenden Härtungsmittel, von denen Divinylsulfon (DE-C 872 153) zu den am längsten bekannten gehört. Einer Anwendung des Divinylsulfons steht seine Toxizität entgegen.

Weiter sind durch DE-C-1 100 942 aromatische Vinylsulfonverbindungen und durch DE-A-1 547 733, DE-B2-1 808 685 und DE-A 2 348 194 Vinylsulfonylalkyl-Verbindungen bekannt geworden, darunter auch solche, die einen heterocyclischen Ring enthalten.

Die bekannten Vinylsulfonverbindungen haben sich als Härtungsmittel in mehrfacher Hinsicht als

nachteilig erwiesen. Sie sind entweder nicht hinreichend wasserlöslich und machen besondere Maßnahmen erforderlich, um ihre Anwendung in fotografischen Gelatineschichten zu ermöglichen, oder sie beeinflussen das Trocknungsverhalten der Schichten in nachteiliger Weise. Andere dieser Verbindungen erhöhen die Viskosität der Gießzusammensetzungen so, daß die Verarbeitung der Gießzusammensetzungen zu Schichten gestört wird. Bekannte Härter des Vinylsulfontyps bewirken auch, insbesondere in farbfotografischen Aufzeichnungsmaterialien, eine Auswanderung fotografischer Zusätze von einer in die andere Schicht, was Farbänderungen und Änderungen der fotografischen Eigenschaften zur Folge hat.

Schließlich sind aus DE-A-2 635 518 als Härtungsmittel Umsetzungsprodukte bekannt, die bei Umsetzungen von Verbindungen mit mindestens 3 Vinylsulfonylgruppen im Molekül mit Verbindungen mit einer wasserlöslichen Gruppe und einer Gruppe, die mit einer Vinylsulfonylgruppe reagieren kann, erhalten werden. Es entstehen dabei z.B. anionische Vinylsulfonylverbindungen.

Diese Verbindungen haben jedoch Nachteile. Sie zeigen in gelatinehaltigen fotografischen Schichten eine starke Nachhärtung, d.h. ihre optimale Wirkung setzt erst nach längerer Lagerzeit des Materials ein. Das hat zur Folge, daß mit zunehmender Dauer der Lagerung eine abnehmende Schichtquellung in Wasser auftritt und daß sich als Folge davon die sensitometrischen Daten des Materials fortlaufend ändern. Außerdem tritt nach Zusatz der bekannten Verbindungen zu gelatinehaltigen Silberhalogenidemulsionen, insbesondere bei pH-Werten um 7, mit zunehmender Digestionsdauer ein Viskositätsanstieg auf, der einen fehlerfreien Beguß nicht mehr zuläßt.

Es ist weiterhin bekannt, daß die Vernetzungsgeschwindigkeit der Gelatine besonders bei den Vinylsulfonylverbindungen von heteroaromatischen Verbindungen im Gegensatz zu den anderen bisher bekannten Vinylsulfonen besonders groß ist. Von Nachteil ist, daß die heteroaromatischen Bis-und Polyvinylsulfonverbindungen in Wasser wenig löslich sind und bei der Anwendung in wäßrigen Gießlösungen ausfallen. Dadurch erhält man eine unregelmäßige Härtung, die im schlimmsten Falle zu Runzelkorn in der Schicht führt.

Der Erfindung liegt die Aufgabe zugrunde, von heteroaromatischen Bis- und Polyvinylsulfonverbindungen gut wasserlösliche Härtungsmittel für proteinhaltige Bindemittel, insbesondere hydrophile Bindemittel, vorzugsweise Gelatine bereitzustellen, die die gleiche Härtungsaktivität besitzen wie die Ausgangsverbindungen und die wegen ihrer Wasserlöslichkeit unter normalen Klimabedingungen keine stärkere Nachhärtung beim Einsatz in fotografischen Schichten zeigen.

Die bekannten wasserlöslichen Additionsprodukte von tertiären Aminen und heteroaromatischen Bis- oder Polyvinylsulfonen haben den Nachteil, daß sie mit anionischen Netzmitteln, die in fotografischen Schichten überwiegend benutzt werden, wasserunlösliche Salze bilden, die zu Fehlern im Beguß führen.

Gegenstand der Erfindung ist ein lichtempfindliches fotografisches Aufzeichnungsmaterial mit mindestens einer mit einem Härtungsmittel gehärteten gelatinehaltigen Schicht, dadurch gekennzeichnet, daß zum Härten eine wasserlösliche sulfatverbindung der allgemeinen Formel (I)

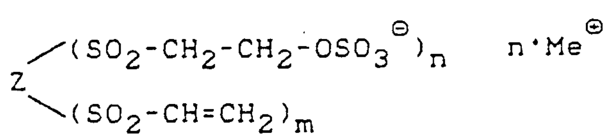

(I)

worin

| | |
|---|---|
| Me | ein Metallkation, wie $Li^+$, $Na^+$, $K^+$ |
| n | eine ganze Zahl > 0, |
| m | eine ganze Zahl ≧ 0 und |
| m + n | 2, und |
| Z | eine der folgenden Formeln |

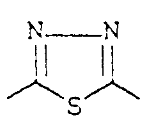

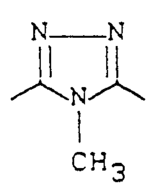

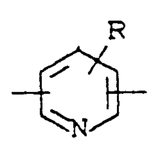

wobei R für $C_1$-$C_4$-Alkyl, Phenyl oder $C_1$-$C_4$-Alkoxy steht, bedeuten Sulfonylethylsulfatgruppenhaltige

Verbindungen von aliphatischen oder nichtaromatisch heterocyclischen Bisoder Polyvinylsulfonen sind bekannt, z.B. aus EP-A-31959 und EP-A-98454 Es gehört aber zu den Eigenschaften dieser Verbindungen, in der fotografischen Schicht langsam zu spalten und erst dann zu härten. Man beobachtet daher eine starke Nachhärtung, d.h. das Maximum der Vernetzungswirkung wird erst nach Tagen oder Wochen erreicht. Das ist für die Herstellung von fotografischen Materialien unerwünscht, da sie möglichst ohne längere Lagerung verkauft werden sollen. Außerdem ändern sich die fotografischen Eigenschaften des Materials fortlaufend entsprechend der höheren Härtung. Weiterhin sind Bis-vinylsulfonylverbindungen des 1,2,4-Triazols und des Thia-3,4-diazols, deren Härtungswirkung unzureichend ist, z.B. aus EP-A-207399 bekannt.

Es ist überraschend, daß die anspruchsgemäßen sulfonylethylsulfatgruppenhaltigen Verbindungen von heterocyclischen aromatischen Verbindungen im Gegensatz zu den bisher bekannten Verbindungen außergewöhnlich schnell mit aminogruppenhaltigen Verbindungen, wie Gelatine unter Vernetzung reagieren. Obwohl sie sehr aktiv sind, sind die wäßrigen Lösungen sehr stabil. Es lassen sich 30 bis 50 gew.-%ige wäßrige Lösungen monatelang bei Raumtemperatur aufbewahren, ohne daß eine Wirkungsverminderung oder eine Polymerisation beobachtet wird.

Die erfindungsgemäßen Härtungsmittel werden aus den hydroxyethylsulfongruppenhaltigen heterocyclisch aromatischen Verbindungen durch Umsetzung mit Chlorsulfonsäure erhalten. Die Hydroxyethylsulfonverbindungen werden wie üblich aus den Hydroxyethylthioverbindungen durch Oxidation mit Wasserstoffperoxid erhalten.

Folgende Verbindungen werden beispielhaft genannt:

H 1

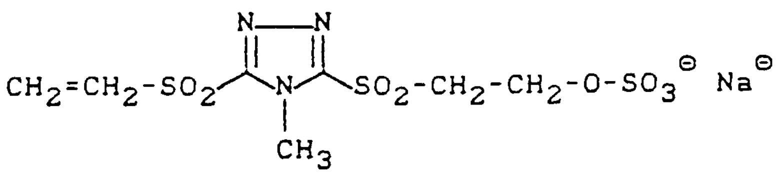

H 2

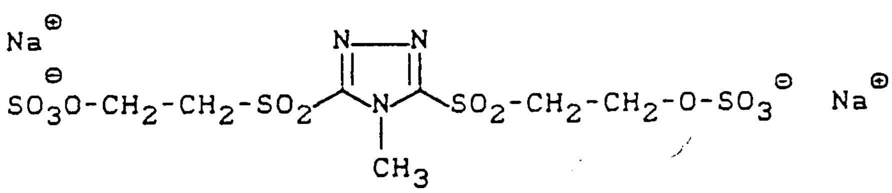

H 3 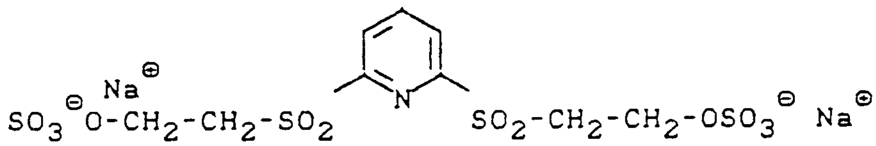

H 4 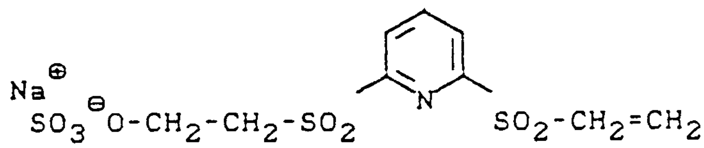

H 5 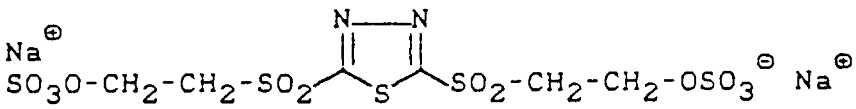

H 6 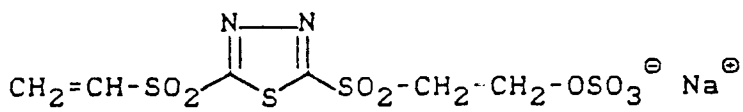

Die Verbindungen werden aus den Hydroxyethylsulfonylverbindungen hergestellt.

Herstellungsbeispiel

6 g 1-N-Methyl-2,5-bis-hydroxylethylsulfonyl-1,3,4-triazol werden in 30 ml abs. Dioxan gerührt. Unter Kühlung bei 10° C werden 9,32 g Chlorsulfonsäure zugetropft. Es entsteht eine gelbliche klare Lösung, die 3 Stunden bei Raumtemperatur gerührt und dann über Nacht bei Raumtemperatur stehen gelassen wird.

Die Mischung wird dann mit Toluol ausgefällt. Das Produkt wird einige Male mit frischem Toluol verrieben. Man löst es dann in Eiswasser und stellt den pH-Wert mit Natriumbicarbonatlösung auf 5. In einem Scheidetrichter wird das anhaftende Toluol abgetrennt und die trübe Lösung mit Bleicherde behandelt. Man erhält eine klare, farblose Lösung. Ausbeute: 150 g 6,6 gew.-%ige Lösung.

Die Verbindung enthält keine Ausgangssubstanz mehr. Die Analyse weist folgende Verbindung aus:

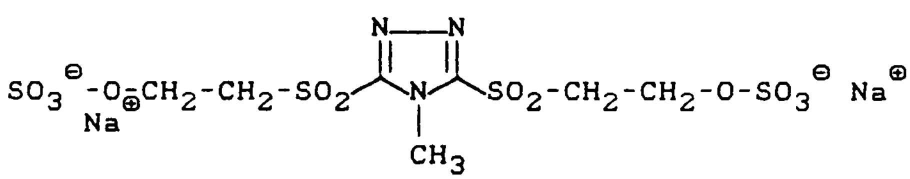

Die in der erfindungsgemäßen Weise zu verwendenden Verbindungen sind im allgemeinen sehr gut in Wasser oder einer Mischung von Wasser mit organischen Lösungsmitteln löslich.

Die gemäß der Erfindung verwendeten Härtungsmittel können der Gießlösung einer herzustellenden Bindemittelschicht, z.B. einer fotografischen Schicht entweder einige Zeit oder unmittelbar vor dem Beguß, zweckmäßigerweise unter Verwendung geeigneter Dosierungseinrichtungen zugesetzt werden. Die Verbindungen können auch einer Übergußlösung zugesetzt werden, die nach Herstellung der eigentlichen Beindemittelschicht als Härtungsschicht auf diese aufgebracht wird. Der fertiggestellte Schichtaufbau kann auch durch eine Lösung der Härtungsmittel gezogen werden und erhält dadurch die notwendige Menge an Härtungsmittel zugeführt. Schließlich lassen sich bei Mehrschichtaufbauten, z.B. bei Colorfilmen und Colorpapier, die Vernetzer der Erfindung über die Zwischenschichten in den Gesamtaufbau einbringen.

Die erfindungsgemäßen Härtungsmittel werden im allgemeinen in einer Menge von 0,01 bis 15 Gew.-% und vorzugsweise von 0,1 bis 5 Gew.-%, bezogen auf das Trockengewicht des Proteins, vorzugsweise der Gelatine in der Beschichtungslösung, angewandt. Der Zeitpunkt der Zugabe zu der Beschichtungslösung ist

nicht kritisch; Silberhalogenidemulsionen wird man den Härter aber zweckmäßigerweise nach der chemischen Reifung zusetzen. pH-kontrollierende Verbindungen wie Bicarbonat oder Natriumacetat lassen sich zusammen mit den erfindungsgemäßen Härtungsmitteln anwenden.

Die erfindungsgemäßen Härtungsmittel können einzeln oder als Gemisch aus zwei oder mehreren erfindungsgemäßen Verbindungen, oder auch zusammen mit anderen bekannten Härtungsmitteln verwendet werden. Geeignete zusätzliche Härtungsmittel sind z.B. Formaldehyd, Glutaraldehyd und ähnliche Aldehydverbindungen, Diacetyl, Cyclopentadion und ähnliche Ketonverbindungen, Bis-(2-chlorethylharnstoff), 2-Hydroxy-4,6-dichlor-1,3,5-triazin und andere Verbindungen, die reaktives Halogen enthalten, wie in US-A-3 288 775, US-A-2 732 303, GB-A-974 723 und GB-A-1 167 207 beschrieben; Divinylsulfon, 5-Acetyl-1,3-diacryloylhexahydro-1,3,5-triazin und andere Verbindungen, die eine reaktive Olefinbindung enthalten, wie in US-A-3 635 718, US-A-3 232 763 und GB-A-994 869 beschrieben; N-Hydroxymethylphthalimid und andere N-Methylolverbindungen, wie in den US-A-2 732 316 und US-A-2 586 168 beschrieben; Isocyanate, wie in US-A-3 103 437 beschrieben; Aziridinverbindungen, wie in US-A-3 017 280 und US-A-2 983 611 beschrieben; Säurederivate, wie in US-A-2 725 294 und US-A-2 725 295 beschrieben; Verbindungen vom Carbodiimidtyp, wie in US-A-3 100 704 beschrieben; Carbomoylpyridiniumsalze wie in DE-A-22 25 230 und DE-A-24 39 551 beschrieben; Carbamoyloxypyridiniumverbindungen wie in DE-A-2 408 814 beschrieben, Verbindungen mit einer Phosphor-Halogen-Bindung wie in JP-A-113 929/83 beschrieben, N-Carbonyloximid-Verbindungen wie in JP-A- 43353/81 beschrieben, N-Sulfonyloxyimido-Verbindungen wie in US-A-4 111 926 beschrieben, Dihydrochinolinverbindungen wie in US-A 4 013 468 beschrieben, 2-Sulfonyloxypyridiniumsalze wie in JP-A-110 762/81 beschrieben, Formamidiniumsalze wie in EP-A-0 162 308 beschrieben, Verbindungen mit zwei oder mehr N-Acyloxyimino-Gruppen im Molekül wie in US-A-4 052 373 beschrieben, Epoxyverbindungen, wie in US-A-3 091 537 beschrieben; Verbindungen vom Isoxazoltyp, wie in US-A 3 321 313 und US-A-3 543 292 beschrieben; Halogencarboxyaldehyde, wie Mucochlorsäure; Dioxanderivate, wie Dihydroxydioxan und Dichlordioxan; und anorganische Härter, wie Chromalaun und Zirkonsulfat. Zusätzlich zu den obigen Härtungsmitteln können die erfindungsgemäßen Härtungsmittel zusammen mit Vorstufen der oben beschriebenen Verbindungen, wie mit Alkalimetallbisulfit-Aldehyd-Addukten, Methylolderivaten von Hydantoin und primären Fettnitroalkoholen, etc., verwendet werden. Wenn die erfindungsgemäßen Härtungsmittel zusammen mit anderen Härtern verwendet werden, kann die Menge an erfindungsgemäßen Härtungsmitteln je nach Bedarf, abhängig von dem Ziel und der Wirkung, ausgewählt werden.

Das Protein der gehärteten Bindemittelschicht gemäß vorliegender Erfindung dient in der Regel als Bindemittel für darin dispergierte reaktive oder nicht reaktive Substanzen, z.B. Farbstoffe oder Verbindungen, die beispielsweise bei Belichtung oder einer sich daran anschließenden Verarbeitung eine Veränderung erfahren und dabei eine bestimmte Wirksamkeit entfalten. Die erfindungsgemäß gehärteten Bindemittelschichten können beispielsweise in Form farbiger Überzüge vorliegen. Das erfindungsgemäß gehärtete Protein eignet sich auch als Bindemittel für diagnostische Zwecke. So können beispielsweise trockenchemische Testmittel, auch Teststreifen genannt, mit einer erfindungsgemäß gehärteten Proteinschicht ausgestattet werden, in die die für die jeweils spezifische Nachweisreaktion erforderlichen Reagentien wie Enzyme, Coenzyme, Farbbildner und dergleichen eingelagert sind. Das gehärtete Protein kann auch in fotografischen oder fotothermografischen ein-oder mehrschichtigen Aufzeichnungsmaterialien Verwendung finden, z.B. als Bindemittel für Silberhalogenid, Farbkuppler und sonstige fotografisch wirksame Substanzen.

Unter fotografischen Schichten sollen im vorliegenden Zusammenhang ganz allgemein Schichten verstanden werden, die im Rahmen fotografischer Aufzeichnungsmaterialien Anwendung finden, beispielsweise lichtempfindliche Silberhalogenidemulsionsschichten, Schutzschichten, Filterschichten, Antihaloschichten, Rückschichten, Bildempfangsschichten oder ganz allgemein fotografische Hilfsschichten.

Als lichtempfindliche gelatinehaltige Emulsionsschichten, für deren Härtung die erfindungsgemäßen Härtungsmittel vorzüglich geeignet sind, seien beispielsweise solche Schichten genannt, die lichtempfindliche Substanzen, insbesondere Silberhalogenid, gegebenenfalls in spektral sensibilisierter Form enthalten. Solche Schichten sind üblicherweise in fotografischen Aufzeichnungsmaterialien für die verschiedensten fotografischen Schwarz-Weiß-oder Farbverfahren, wie Negativ-, Positiv-, Diffusionsübertragungs- oder Druckverfahren, enthalten. Als besonders vorteilhaft haben sich die erfindungsgemäßen Härtungsmittel für die Härtung fotografischer Schichtverbände erwiesen, die zur Durchführung farbfotografischer Prozesse bestimmt sind, z.B. solcher, die Farbkuppler oder andere farbgebende Verbindungen enthalten oder solcher, die zur Behandlung mit farbkupplerhaltigen Lösungen bestimmt sind.

Die Wirkung der erfindungsgemäßen Härtungsmittel wird durch die üblichen fotografischen Zusätze nicht beeinträchtigt. Ebenso sind die Härtungsmittel indifferent gegenüber fotografisch wirksamen Substanzen, wie wasserlöslichen oder emulgierten wasserunlöslichen Farbkomponenten, Stabilisatoren, Sensibilisatoren und dergleichen. Sie üben ferner keinen nachteiligen Einfluß auf die lichtempfindliche Silberhalogenidemulsion aus.

Das als lichtempfindlicher Bestandteil in dem lichtempfindlichen Silberhalogenid kann als Halogenid Chlorid, Bromid und Iodid bzw. Mischungen davon enthalten. Beispielsweise kann der Halogenidanteil wenigstens einer Schicht zu 0 bis 12 mol-% aus Iodid, zu 0 bis 50 mol-% aus Chlorid und zu 50 bis 100 mol-% aus Bromid bestehen. Es kann sich um überwiegend kompakte Kristalle handeln, die z.B. kubisch oder oktaedrisch sind oder Übergangsformen aufweisen können. Im wesentlichen weisen sie eine Dicke von mehr als 0,2 µm auf. Das durchschnittliche Verhältnis von Durchmesser zu Dicke ist bevorzugt kleiner als 8:1, wobei gilt, daß der Durchmesser eines Kornes definiert ist als der Durchmesser eines Kreises mit einem Kreisinhalt entsprechend der projizierten Fläche des Kornes. Die Schichten können aber auch tafelförmige Silberhalogenidkristalle aufweisen, bei denen das Verhältnis von Durchmesser zu Dicke größer als 8:1 ist. Zu verweisen ist auf Research Disclosure 22 534 (Januar 1983). Bei den Emulsionen kann es sich um heterodisperse oder auch um monodisperse Emulsionen handeln, die bevorzugt eine mittlere Korngröße von 0,3 µm bis 1,2 µm aufweisen. Die Silberhalogenidkörner können auch einen geschichteten Kornaufbau aufweisen. Die Emulsionen können außer dem Silberhalogenid auch organische Silbersalze enthalten, z.B. Silberbenzotriazolat oder Silberbehenat.

Die Emulsionen können in der üblichen Weise chemisch und oder spektral sensibilisiert sein; sie können auch durch geeignete Zusätze stabilisiert sein. Geeignete chemische Sensibilisatoren, spektrale Sensibilisierungsfarbstoffe und Stabilisatoren sind beispielsweise in Research Disclosure 17643 beschrieben; verwiesen wird insbesondere auf die Kapitel III, IV und VI.

Das erfindungsgemäß hauptsächlich verwendete Bindemittel ist ein proteinartiges Bindemittel, insbesondere Gelatine. Wesentliches Merkmal dieses Bindemittels ist die Anwesenheit von funktionellen Gruppen, mit denen die Vinylsulfonylgruppen des erfindungsgemäßen Härtungsmittels reagieren können, insbesondere Aminogruppen. Das proteinartige Bindemittel kann teilweise z.B. durch partielle Acylierung modifiziert sein oder durch andere natürliche oder synthetische Bindemittel ersetzt sein, solange eine ausreichende Reaktionsfähigkeit mit dem erfindungsgemäßen Härtungsmittel erhalten bleibt. Begußhilfsmittel und Weichmacher können verwendet werden. Verwiesen wird auf Research Disclosure 17 643, insbesondere Kapitel IX, XI und XII.

Die Bindemittelschicht kann fotografisch inerte Teilchen anorganischer oder organischer Natur enthalten, z.B. als Mattierungsmittel oder als sogenannte Abstandshalter. Solche Teilchen können aus einem organischen Polymer bestehen. Verwiesen wird beispielsweise auf DE-A-33 31 542, DE-A-34 24 893, sowie auf Research Disclosure 17 643, Kapitel XVI.

Zur Verbesserung des Begusses werden den Schichten Netzmittel - bevorzugt anionische Netzmittel - zugesetzt. Darunter versteht man oberflächenaktive Verbindungen mit $-SO_3^{\ominus}$, $-OSO_3^{\ominus}$ und $-COO^{\ominus}$ Gruppen. Es werden Alkylsulfonate

$R\text{-}CH_2\text{-}SO_3^{\ominus}\ Me^{\oplus}$ $\quad R = C_{10}\text{-}C_{17}$

Alkylsulfate

$R\text{-}CH_2\text{-}O\text{-}SO_3^{\ominus}\ Me^{\oplus}$ $\quad R = C_{10}\text{-}C_{17}$

Bernsteinsäureester-sulfonate

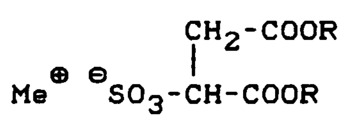

$R = C_5\text{-}C_{10}$

Alkylmethyltaurin, Mono- und Dialkylnaphthalinsulfonate und Perfluoralkylsulfonate verwendet.

Als Kationen kommen z.B. $Li^{\oplus}$, $Na^{\oplus}$, $K^{\oplus}$ infrage.

Diese können in Kombination mit nichtionischen und amphoteren Netzmitteln eingesetzt werden.

Farbfotografische Aufzeichnungsmaterialen enthalten üblicherweise mindestens je eine Silberhalogenidemulsionsschicht für die Aufzeichnung von Licht jedes der drei Spektralbereiche Rot, Grün und Blau. Zu diesem Zweck sind die lichtempfindlichen Schichten in bekannter Weise durch geeignete Sensibilisierungsfarbstoffe spektral sensibilisiert. Blauempfindliche Silberhalogenidemulsionsschichten müssen nicht notwendigerweise einen Spektralsensibilisator enthalten, da für die Aufzeichnung von blauem Licht in vielen Fällen die Eigenempfindlichkeit des Silberhalogenids ausreicht.

Jede der genannten lichtempfindlichen Schichten kann aus einer einzigen Schicht bestehen oder in bekannter Weise, z.B. bei der sogenannten Doppelschichtanordnung, auch zwei oder auch mehr Silberhalogenidemulsionsteilschichten umfassen (DE-C-1 121 470). Üblicherweise sind rotempfindliche Silberhalogenidemulsionsschichten dem Schichtträger näher angeordnet als grünempfindliche Silberhalogenidemulsionsschichten und diese wiederum näher als blauempfindliche, wobei sich im allgemeinen zwischen grünempfindlichen Schichten und blauempfindlichen Schichten eine nicht lichtempfindliche gelbe Filterschicht befindet. Es sind aber auch andere Anordnungen denkbar. Zwischen Schichten unterschiedlicher Spektralempfindlichkeit ist in der Regel eine nicht lichtempfindliche Zwischneschicht angeordnet, die Mittel zur Unterbindung der Fehldiffusion von Entwickleroxidationsprodukten enthalten kann. Falls mehrere Silberhalogenidemulsionsschichten gleicher Spektralempfindlichkeit vorhanden sind, können diese einander unmittelbar benachbart sein oder so angeordnet sein, daß sich zwischen ihnen eine lichtempfindliche Schicht mit anderer Spektralempfindlichkeit befindet (DE-A-1 958 709, DE-A-2 530 645, DE-A-2 622 922).

Farbfotografische Aufzeichnungsmaterialien zur Herstellung mehrfarbiger Bilder enthalten üblicherweise in räumlicher und spektraler Zuordnung zu den Silberhalogenidemulsionsschichten unterschiedlicher Spektralempfindlichkeit farbgebende Verbindungen, z.B. Farbkuppler, zur Erzeugung der unterschiedlichen Teilfarbenbilder Cyan, Purpur und Gelb.

Unter räumlicher Zuordnung ist dabei zu verstehen, daß der Farbkuppler sich in einer solchen räumlichen Beziehung zu der Silberhalogenidemulsionsschicht befindet, daß eine Wechselwirkung zwischen ihnen möglich ist, die eine bildgemäße Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nichtlichtempfindlichen Bindemittelschicht.

Unter spektraler Zuordnung ist zu verstehen, daß die Spektralempfindlichkeit jeder der lichtempfindlichen Silberhalogendemulsionsschichten und die Farbe des aus dem jeweils räumlich zugeordneten Farbkuppler erzeugten Teilfarbenbildes in einer bestimmten Beziehung zueinander stehen, wobei jeder der Spektralempfindlichkeiten (Rot, Grün, Blau) eine andere Farbe des betreffenden Teilfarbenbildes (im allgemeinen z.B. die Farben Cyan, Purpur bzw. Gelb in dieser Reihenfolge) zugeordnet ist.

Jeder der unterschiedlich spektral sensibilisierten Silberhalogenidemulsionsschichten kann ein oder können auch mehrere Farbkuppler zugeordnet sein. Wenn mehrere Silberhalogenidemulsionsschichten gleicher Spektralempfindlichkeit vorhanden sind, kann jede von ihnen einen Farbkuppler enthalten, wobei diese Farbkuppler nicht notwendigerweise identisch zu sein brauchen. Sie sollen lediglich bei der Farbentwicklung wenigstens annähernd die gleiche Farbe ergeben, normalerweise eine Farbe, die komplementär ist zu der Farbe des Lichtes, für das die betreffenden Silberhalogenidemulsionsschichten überwiegend empfindlich sind.

Rotempfindlichen Silberhalogenidemulsionsschichten ist folglich bei bevorzugten Ausführungsformen mindestens ein nichtdiffundierender Farbkuppler zur Erzeugung des blaugrünen Teilfarbenbildes zugeordnet, in der Regel ein Kuppler vom Phenol- oder $\alpha$-Naphtholtyp. Grünempfindlichen Silberhalogenidemulsionsschichten ist mindestens ein nichtdiffundierender Farbkuppler zur Erzeugung des purpurnen Teilfarbenbildes zugeordnet, wobei üblicherweise Farbkuppler vom Typ des 5-Pyrazolons, des Indazolons oder des Pyrazoloazols Verwendung finden. Blauempfindlichen Silberhalogenidemulsionsschichten schließlich ist mindestens ein nichtdiffundierender Farbkuppler zur Erzeugung des gelben Teilfarbenbildes zugeordnet, in der Regel ein Farbkuppler mit einer offenkettigen Ketomethylengruppierung. Farbkuppler dieser Art sind in großer Zahl bekannt und in einer Vielzahl von Patentschriften beschrieben.

Beispielhaft sei hier auf die Veröffentlichungen "Farbkuppler" von W. PELZ in "Mitteilungen aus den Forschungslaboratorien der Agfa, Leverkusen/München", Band III, Seite 111 (1961) und von K. VENKATARAMAN in "The Chemistry of Synthetic Dyes", Vol. 4, 341 bis 387, Academic Press (1971), verwiesen, sowie auf Research Disclosure 17 643, Kapitel VIII.

Bei den Farbkupplern kann es sich sowohl um übliche 4-Äquivalentkuppler handeln als auch um 2-Äquivalentkuppler, bei denen zur Farberzeugung eine geringere Menge Silberhalogenid erforderlich ist. 2-Äquivalentkuppler leiten sich bekanntlich von den 4-Äquivalentkupplern dadurch ab, daß sie in der Kupplungsstelle einen Substituenten enthalten, der bei der Kupplung abgespalten wird. Zu den 2-Äquivalentkupplern sind sowohl solche zu rechnen, die praktisch farblos sind, als auch solche, die eine intensive Eigenfarbe aufweisen, die bei der Farbkupplung verschwindet bzw. durch die Farbe des erzeugten Bildfarbstoffes ersetzt wird. Letztere Kuppler können ebenfalls zusätzlich in den lichtempfindlichen Silberhalogenidemulsionsschichten vorhanden sein und dort als Maskenkuppler zur Kompensierung der unerwünschten Nebendichten der Bildfarbstoffe dienen. Zu den 2-Äquivalentkupplern sind aber auch die bekannten Weißkuppler zu rechnen, die jedoch bei Reaktion mit Farbentwickleroxidationsprodukten keinen Farbstoff ergeben. Zu den 2-Äquivalentkupplern sind ferner solche Kuppler zu rechnen, die in der Kupplungsstelle einen abspaltbaren Rest enthalten, der bei Reaktion mit Farbentwickleroxidationsprodukten in Freiheit gesetzt wird, und dabei entweder direkt, oder nachdem aus dem abgespaltenen Rest eine oder

mehrere Gruppen abgespaltet worden sind (z.B. DE-A-27 03 145, DE A-28 55 697, DE-A-31 05 026, DE-A-33 19 428), eine bestimmte erwünschte fotografische Wirksamkeit entfaltet, z.B. als Entwicklungsinhibitor oder -accelerator. Beispiele für solche 2-Äquivalentkuppler sind die bekannten DIR-Kuppler wie auch DAR- bzw. FAR-Kuppler. Der abgespaltbare Rest kann auch ein Ballastrest sein, so daß bei der Reaktion mit Farbentwickleroxidationsprodukten Kupplungsprodukte z.B. Farbstoffe erhalten werden können, die diffusionsfähig sind oder zumindest eine schwache bzw. eingeschränkte Beweglichkeit aufweisen.

Unter einer schwachen bzw. eingeschränkten Beweglichkeit ist eine Beweglichkeit zu verstehen, die so bemessen ist, daß die Konturen der bei der chromogenen Entwicklung gebildeten diskreten Farbstoffflecken verlaufen und ineinander verschmiert werden. Dieses Ausmaß der Beweglichkeit ist einerseits zu unterscheiden von dem üblichen Fall der völligen Unbeweglichkeit in fotografischen Schichten, der in der herkömmlichen fotografischen Aufzeichnungsmaterialien für die Farbkuppler bzw. die daraus hergestellten Farbstoffe angestrebt wird um eine möglichst hohe Schärfe zu erzielen, und andererseits von dem Fall der völligen Beweglichkeit der Farbstoffe, der beispielsweise bei Farbdiffusionsverfahren angestrebt wird. Die letztgenannten Farbstoffe verfügen meist über mindestens eine Gruppe, die sie im alkalischen Medium löslich macht. Das Ausmaß der erfindungsgemäß angestrebten schwachen Beweglichkeit kann gesteuert werden durch Variation von Substituenten um beispielsweise die Löslichkeit im organischen Medium des Ölbildners oder die Affinität zur Bindemittelmatrix in gezielter Weise zu beeinflussen.

Hochmolekulare Farbkuppler sind beispielsweise beschrieben in DE-C-1 297 417, DE-A-24 07 569, DE-A-31 48 125, DE-A-32 17 200, DE-A-33 20 079, DE-A-33 24 932, DE-A-33 31 743, DE-A-33 40 376, EP-A-27 284, US-A-4 080 211. Die hochmolekularen Farbkuppler werden in der Regel durch Polymerisation von ethylenisch ungesättigten monomeren Farbkupplern hergestellt. Sie können aber auch durch Polyaddition oder Polykondensation erhalten werden.

Die Bindemittelschichten können Filter- und Antihalofarbstoffe enthalten, z.B. Oxonolfarbstoffe wie beschrieben in US-A-2 274 782, US-A-2 611 696, FR-A-1 290 430,
GB-A 1 177 429, DE-A-1 572 256, DE-A-22 59 746, DE-A-23 21 470, US-A-3 984 246, Styrylfarbstoffe wie beschrieben in US-A-2 036 546, DE-B-1 014 430, DE-B-1-028 425, DE-B-1 112 801, DE-B-1 104 335, Azofarbstoffe wie beschrieben in DE-B-1 103 135, DE-B-1 182 067, GB-A-1 122 298, DE-A-1 547 646, Triphenylmethanfarbstoffe wie beschrieben in DE-B-1 008 114, Anthrachinonfarbstoffe wie beschrieben in US-A-2 865 752 oder Merocyanine wie beschrieben in GB-A-1 030 392 oder US-A-4 366 221. Verwiesen wird auch auf Research Disclosure 17 643, Kapitel VIII.

Die Bindemittelschichten können UV-Absorber enthalten, gegebenenfalls auch in hochmolekularer Form. Verwiesen wird auf DE-A-35 01 722, DE-A-35 05 423, DE-A-35 31 383, EP-A-0 027 242, EP-A-0 057 160, sowie auf Research Disclosure 17 643, Kapitel VIII und Research Disclosure 18 716, insbesondere Seite 650, linke Spalte.

Die Bindemittelschichten können Farbstoffstabilisatoren enthalten, wie beschrieben in DE-A-35 01 722, EP-A-0 011 051, EP-A-0 026 742, EP-A-0 069 070, EP-A-0 098 241, EP-A-0 114 028, EP-A-0 114 029, sowie Research Disclosure 17 643, Kapitel VII, insbesondere Abschnitt J.

Die Bindemittelschichten können optische Aufheller oder Weißtöner enthalten. Verwiesen wird beispielsweise auf Research Disclosure 17 643, Kapitel V.

Die Bindemittelschichten können sogenannte Scavenger-Verbindungen enthalten, d.h. Verbindungen die mit Entwickleroxidationsprodukten zu reagieren und diese an der Diffusion in Nachbarschichten zu hindern vermögen. Verwiesen wird beispielsweise auf EP-A-0 098 072, EP-A-0 124 877 EP-A-0 069 070, US-A-4 366 226 und EP-A-0 125 522, sowie auf Research Disclosure 17 643, insbesondere Kapitel VII, Abschnitt I, Research Disclosure 17 842 (Februar 1979) und Research Disclosure 18 716 (November 1979) insbesondere Seite 650.

Die Zugabe der einzubringenden Verbindungen kann in der Weise erfolgen, daß zunächst von der betreffenden Verbindung eine Lösung oder ein Dispergat hergestellt und dann der Gießlösung zugefügt wird. Das Lösungs- oder Dispergiermittel richtet sich nach den jeweiligen Bedarf. Hydrophobe Verbindungen können unter Verwendung von hochsiedenden Lösungsmitteln, sogenannten Ölbildnern, in die Gießlösung eingebracht werden. Entsprechende Methoden sind beispielsweise beschrieben in US-A-2 322 027, DE-A-1 722 192 und EP-A-0 043 037. Die Verbindungen können auch in Form beladener Latices in die Gießlösung eingebracht werden. Verweisen wird beispielsweise auf DE-A-25 41 230, DE-A-25 41 274, DE-A-28 35 856, EP-A-0 014 921, EP-A-0 069 671, EP-A-0 130 115, US-A-4 291 113.

Die Bindemittelschichten können des weiteren Mittel enthalten, die mit Aldehyden, insbesondere mit Formaldehyd zu reagieren vermögen, sogenannte Aldehydentfernungsmittel, oder Verbindungen, die andere eingelagerte Verbindungen, insbesondere andere Farbkuppler gegen den schädlichen Einfluß von Aldehyden zu schützen vermögen. Zu solchen Aldehydentfernungsmitteln gehören beispielsweise N,N'-Ethylenharnstoff, 2,3-Dihydroxynaphthalin oder Dimedon. Verwiesen wird beispielsweise auf DE-A-1 772

816.

Zur Verarbeitung wird das erfindungsgemäße Aufzeichnungsmaterial mit einer Farbentwicklerverbindung entwickelt. Als Farbentwicklerverbindung lassen sich sämtliche Entwicklerverbindungen verwenden, die die Fähigkeit besitzen in Form ihres Oxidationsproduktes mit Farbkupplern zu Azomethin- bzw. Indochinonfarbstoffen zu reagieren. Geeignete Farbentwicklerverbindungen sind aromatische mindestens eine primäre Aminogruppe enthaltende Verbindungen vom p-Phenylendiamintyp, beispielsweise N,N-Dialkyl-p-phenylendiamine, wie N,N-Diethyl-p-phenylendiamin, 1-(N-ethyl-N-methylsulfonamidoethyl)-3-methyl-p-phenylendiamin, 1-(N-ethyl-N-hydroxyethyl-3-methyl-p-phenylendiamin und 1-(N-ethyl-N-methoxyethyl)-3-methyl-p-phenylendiamin. Weitere brauchbare Farbentwickler sind beispielsweise beschrieben in J. Amer. Chem. Soc. 73. 3106 (1951) und in G. Haist, Modern Photographie Processing, 1979, John Wiley and Sons, New York, Seite 545 ff.

Nach der Farbentwicklung wird das Material üblicherweise gebleicht und fixiert. Bleichung und Fixierung können getrennt voneinander oder auch zusammen durchgeführt werden. Als Bleichmittel können die üblichen Verbindungen verwendet werden, z.B. $Fe^{3+}$-Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren, insbesondere z.B. Ethylendiamintetraessigsäure, Nitrilotriessigsäure, Iminodiessigsäure, N-Hydroxyethylethylendiamintriessigsäure, Alkyliminodicarbonsäuren und von entsprechenden Phosphonsäuren. Geeignet als Bleichmittel sind weiterhin Persulfate.

Günstige Ergebnisse können erhalten werden bei Verwendung eines wäßrigen Schlußbades, das keinen oder nur wenig Formaldehyd enthält.

Beispiel 1

Auf einen mit einer Haftschicht versehenen Cellulosetriacetat-Schichtträger wurden nacheinander folgende Schichten aufgetragen (die angegebenen Mengen beziehen sich auf 1 $m^2$).

1. Eine Lichthofschutzschicht mit 4 g Gelatine und 0,7 g kolloidalem schwarzen Silber,

2. eine 6 µm dicke rotempfindliche Schicht mit 35 mmol Silberbromidiodid (5 mol-% AgI), 4 mmol eines Blaugrünkupplers der Formel

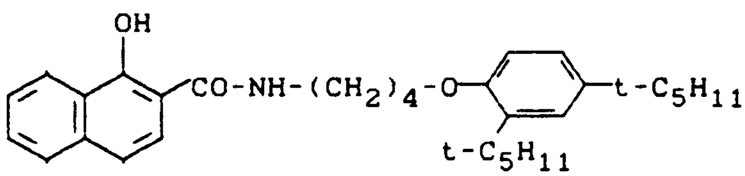

6 g Gelatine und 0,3 g dibutylnaphthalinsulfonsaures Natrium,

3. eine 0,5 µm dicke Gelatinezwischenschicht,

4. eine 6 µm dicke grünempfindliche Schicht mit 30 mmol Silberbromidiodid (5 mol % AgI), 1,3 mmol eines Purpurkupplers der Formel

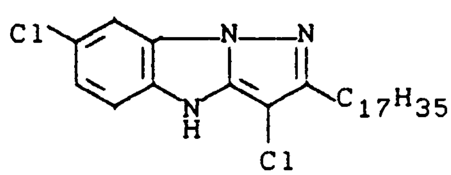

5 g Gelatine und 0,2 g dibutylnaphthalinsulfonsaures Natrium,

5. eine 0,5 µm dicke Gelatinezwischenschicht,

6. eine Gelbfilterschicht mit 1,5 g Gelatine und 0,2 g kolloidalem gelben Silber,

7. eine 6 µm dicke blauempfindliche Schicht mit 13 mmol Silberbromidiodid (5 mol-% AgI), 2 mmol eines Gelbkupplers der Formel

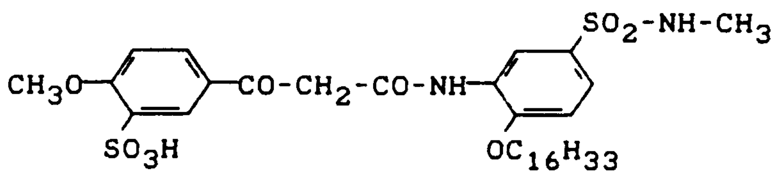

und 5 g Gelatine und

8. eine 1 µm dicke Gelatineschicht.

Alle Gießlösungen enthielten als Gießnetzmittel Sulfobernsteinsäureoctylester als Na-Salz.

Der Schichtverband wurde danach getrocknet.

Der so hergestellte fotografische Film diente im folgenden als Vergleichsmaterial.

Die Herstellung des Films wurde wiederholt, wobei den einzelnen Schichten je Filmprobe eines der erfindungsgemäßen Härtungsmittel H-2 und H-3 bzw. zum Vergleich das nicht erfindungsgemäße Härtungsmittel V-1 in einer Menge von 0,0075 Mol/100 g Gelatine zugesetzt wurden.

Das nicht erfindungsgemäße Härtungsmittel V-1 hat die Formel

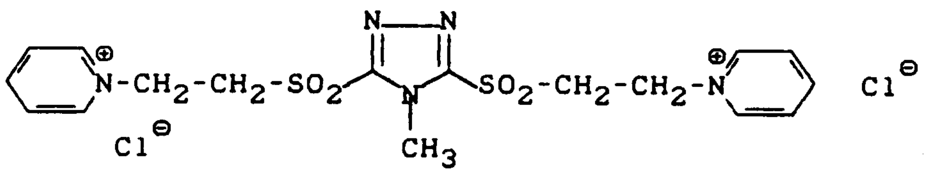

Die in der beschriebenen Weise gehärteten Proben wurden nun nach den anschließend beschriebenen Methoden geprüft. Die Ergebnisse sind in Tabelle 1 dargestellt.

Die Härtung der fotgrafischen Schichten wurde mit Hilfe des Schmelzpunktes der Schichten bestimmt, der sich wie folgt ermitteln läßt.

Der auf eine Unterlage vergossene Schichtverband wird zur Hälfte in Wasser getaucht, das kontinuierlich bis 100 °C erwärmt wird. Die Temperatur, bei der die Schicht von der Unterlage abläuft (Schlierenbildung), wird als Schmelzpunkt bzw. Abschmelzpunkt bezeichnet. Bei diesem Meßverfahren zeigen ungehärtete Protein- bzw. Colorschichten in keinem Falle eine Schmelzpunkterhöhung. Der Abschmelzpunkt liegt unter diesen Bedingungen bei 30 bis 35 °C.

Zur Bestimmung der Wasseraufnahme wurde der Prüfling in einem herkömmlichen Farbentwicklungsprozeß als Schwarzblatt entwickelt und nach dem Schlußbad nach Abstreifen des überschüssigen Wassers gewogen. Dann wurde die Probe getrocknet und erneut gewogen. Die Gewichtsdifferenz ergibt von der Fläche des Prüflings auf 1 $m^2$ umgerechnet die Wasseraufnahme pro $m^2$.

Die Quellung wurde nach 10 min Behandlung eines Probestreifens in destilliertem Wasser bei 22 °C gravimetrisch gemessen. Sie wird durch den Quellfaktor chrakterisiert:

$$\frac{\text{Schichtgewicht naß}}{\text{Schichtgewicht trocken}} = \text{Quellfaktor}$$

Zur Bestimmung der Naßkratzfestigkeit wurde eine Metallspitze definierter Größe über die nasse Schicht geführt und mit zunehmendem Gewicht belastet. Die Naßkratzfestigkeit wird durch das Gewicht angegeben, bei dem die Spitze eine sichtbare Kratzspur auf der Schicht hinterläßt. Ein hohes Gewicht entspricht einer hohen Naßkratzfestigkeit.

Das Runzelkorn wird nach Quellung (10 Minuten) in Wasser bei 22 °C mit der Lupe (8 x) beurteilt. Eine durch die Unlöslichkeit des Härtungsmittels in wäßriger Lösung hervorgerufene ungleichmäßige Härtung der gelatinehaltigen Schichten führt zu einer örtlich verschiedenen Vertikal-und Horizontalquellung und damit zu einer ungleichmäßigen Oberfläche (Runzelkorn).

T a b e l l e 1

| | | Lagerung 36 h bei 57° C, 34 % rel. LF. | | | Lagerung 3d bei 23° C Feuchtigkeitsausschluß | Lagerung 7 d bei 36° C, 80 % rel. LF. | | | |
|---|---|---|---|---|---|---|---|---|---|
| Proben Nr. | Härtungsmittel | Schmelzpunkt der Schicht [° C] | Quellfaktor | Naßkratzfestigkeit [N] | Schmelzpunkt der Schicht (° C) | Schmelzpunkt der Schicht [° C] | Quellfaktor | Naßkratzfestigkeit [N] | Runzelkorn |
| 1 | - | 40 | 6-8 | - | 40 | 40 | 6-8 | - | - |
| 2 | H-2 | 100 | 2,5 | 5,5 | 100 | 100 | 2,4 | 6,0 | - |
| 3 | V-1 | 100 | 3,1 | <3,0 | 100 | 100 | 3,2 | 3,5 | starkes Runzelkorn |

Es wurden bei den Proben 1 und 2 keine Veränderungen der fotografischen Eigenschaften der Proben, wie Empfindlichkeit, Schleier und Gradation beobachtet. Probe 3 zeigte starkes Runzelkorn und Schichtverwerfungen.

Aus Tabelle 1 ist ersichtlich, daß die erfindungsgemäße Verbindung H-2 einwandfreie und homogengehärtete Schichten liefert, während die Vergleichsverbindung V-1 mit den anionischen Netzmitteln in der Schicht nicht verträglich ist und durch Koagulation Runzelkorn erzeugt.

Beispiel 2

Ein Farbaufsichtsmaterial wurde hergestellt, indem auf eine polyethylen-kaschierte und mit einer Haftschicht versehenen Papierunterlage nacheinander folgende Schichten aufgetragen wurden, wobei die Emulsionsschichten die üblichen Zusätze an Netzmittel, Stabilisatoren usw. enthielten:

1. Als Unterguß eine 4 µm dicke blauempfindliche Silberhalogenidemulsionsschicht, die pro kg Emulsion 25,4 g Silberchloridbromid (12 mol-% AgCl), 80 g Gelatine, 0,9 g dibutylnaphthalisulfonsaures Natrium und 34 g der Gelbkomponente

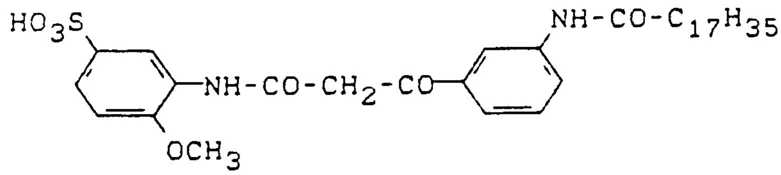

enthält.

2. als Zwischenschicht eine 1 µm dicke Gelatineschicht,

3. als Mittelguß eine 4 µm dicke grünempfindliche Silberhalogenidemulsionsschicht,die pro kg Emulsion 22 g Silberchloridbromid (77 mol-% AgCl), 80 g Gelatine, 13 g der Purpurkomponente,

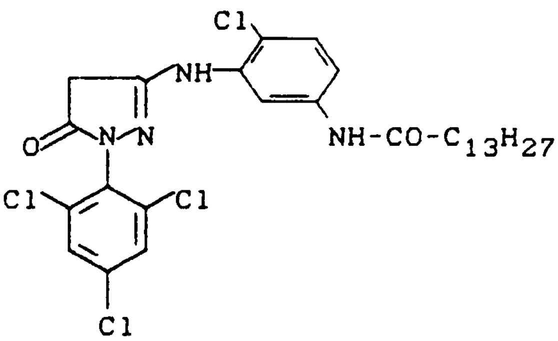

und 0,65 g dibutylnaphthalinsulfonsaures Natrium enthält,

4. eine 1 µm dicke Zwischenschicht wie unter 2. angegeben,

5. als Oberguß eine 4 µm dicke rotempfindliche Silberhalogenidemulsionsschicht, die pro kg Emulsion 23 g Silberchloridbromid (80 mol-% AgCl), 80 g Gelatine, 15,6 g der Blaugrünkomponente

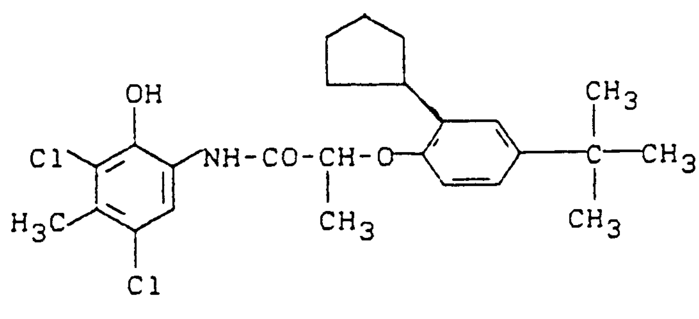

und 0,6 g dibutylnaphthalinsulfonsaures Natrium enthält,

6. eine 1 µm dicke Schutzschicht aus Gelatine.

Auf das getrocknete Schichtpaket wurden wäßrige Lösungen der Verbindung H-2 und des nicht erfindungsgemäßen Härtungsmittels V-2 (jeweils 1/200 mol pro 100 ml) aufgetragen und der Schichtverband danach getrocknet. Die Schichten wurden auf Vernetzung nach Klima- und Tropenlagerung untersucht.

V-2:

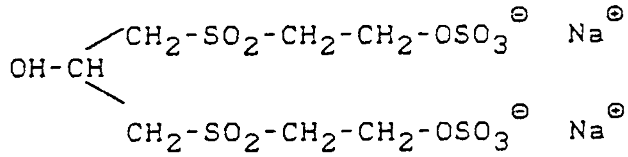

Die Ergebnisse sind in Tabelle 2 dargestellt.

T a b e l l e 2

| Proben Nr. | Härtungsmittel | Lagerung 36 h bei 57°C, 34 % rel. LF. – Schmelzpunkt der Schicht [°C] | Lagerung 36 h bei 57°C, 34 % rel. LF. – Quellfaktor | Lagerung 36 h bei 57°C, 34 % rel. LF. – Naßkratzfestigkeit [N] | Lagerung 3d bei 23°C Feuchtigkeitsausschluß – Schmelzpunkt der Schicht [°C] | Lagerung 7 d bei 36°C, 80 % rel. LF. – Schmelzpunkt der Schicht [°C] | Lagerung 7 d bei 36°C, 80 % rel. LF. – Quellfaktor | Lagerung 7 d bei 36°C, 80 % rel. LF. – Naßkratzfestigkeit [N] |
|---|---|---|---|---|---|---|---|---|
| 1 | H-2 | 100 | 3,8 | 4,5 | 100 | 100 | 3,5 | 5,0 |
| 2 | V-2 | 40 | 5-6 | < 2,0 | 40 | 100 | 4,2 | <2,0 |

Aus der Tabelle 2 ist zu ersehen, daß die Vergleichssubstanz V-2 wesentlich langsamer härtet als das erfindungsgemäße Härtungsmittel (hohe Nachhärtung).

Der gesamte Schichtverband wird durch die eindiffundierenden erfindungsgemäßen Verbindungen bereits nach der Klimalagerung kochfest gehärtet. Die Schichten haben einwandfreie Grenzflächen und sind ohne Runzelkorn.

Die Vergleichsverbindung V-2 vernetzt erst nach Tropenlagerung. Dadurch erhält man eine starke Nachhärtung bei der Lagerung, wodurch sich die fotografischen Eigenschaften im Laufe der Zeit verändern. (Abnahme der Gradation, Verringerung der maximalen Dichte).

Man findet bei den erfindungsgemäßen Härtern keine Abnahme der Härtungsintensität in Abhängigkeit vom Abstand von der obersten Schicht (Auftragsschicht mit Härtungsmittel).

Nach der farbfotografischen Verarbeitung in den üblichen Verarbeitungsbädern werden Schichten mit vergleichbaren fotografischen Werten hinsichtlich Empfindlichkeit, Schleier, Gradation erhalten. Das Härtungssystem der Erfindung verhält sich in dieser Anwendungsform gegenüber der Emulsion, den Farbkupplern und den angewandten anionischen Netzmitteln inert.

Beispiel 3

Auf einem Celluloseacetat-Träger wurde eine gelatinehaltige AgBr/AgI-Emulsion mit entsprechender Sensibilisierung und Stabilisierung mit einer 2 µm dicken Schutzschicht angebracht. Das Schichtpaket wurde mit einer Übergußlösung mit 1,5 Gew.-% Gelatine mit 0,1 Gew.-% Netzmittel der Formel

$$C_8F_{17}SO_3^{\ominus}\ \overset{\oplus}{N}(C_2H_5)_4$$

und jeweils mit dem Härtungsmittel in einer derartigen Menge überschichtet, daß jeweils 0,08 Mol Härtungsmittel auf 1 kg Gelatine einwirkten.

Die Schichten wurden getrocknet und jeweils 3 Tage bei Raumtemperatur unter Feuchtigkeitsausschluß und 3 Tage bei 57° C und 34 % rel. Luftfeuchtigkeit gelagert.

Anschließend wurden die Schichtschmelzpunkte, der Quellfaktor in Wasser von 22° C und die Naßfestigkeit im Schwarzweißentwickler bei 38° C bestimmt.

Als Vergleichshärtungsmittel dienten V-1 und V-3, wobei V-3 die folgende Formel hat:

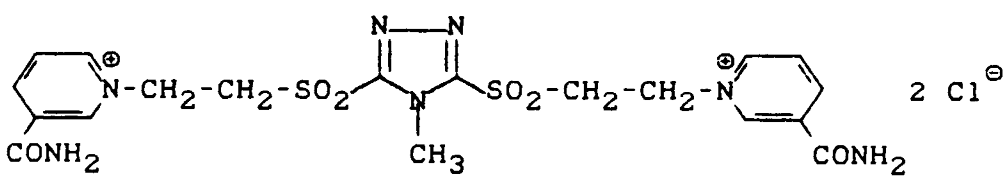

Tabelle 3

| Verbindung 0,08 Mol/kg Gelatine | Werte nach Lagerung 3 Tage / 22° C Feuchtigkeitsausschluß | | | | Lagerung 3 Tage / 57° C 34 % rel. LF. | |
|---|---|---|---|---|---|---|
| | Dauer der Beständigkeit gegen kochendes Wasser (Min) | Quellfaktor | Naßkratzfestigkeit SW-Entwickler 38° C | Runzelkorn | Quellfaktor | Naßkratzfestigkeit SW-Entwickler 38° C |
| V-1 | 7 | 3,0 | 2,0 | starkes Runzelkorn | 2,9 | 2,5 |
| V-3 | 6 | 2,8 | 2,0 | " | 3,0 | 2,5 |
| H-1 | 10 | 2,6 | 3,0 | kein Runzelkorn | 2,7 | 5,0 |
| H-2 | 10 | 3,0 | 3,5 | " | 3,2 | 5,0 |
| H-3 | 10 | 5,4 | 1,5 | " | 3,0 | 3,5 |

Aus der Tabelle 3 ist zu ersehen, daß die erfindungsgemäßen Verbindungen auch im Falle einer etwas geringeren Härtungswirkung kein Schichtrunzelkorn zeigen und daß in allen Fällen eine völlig gleichmäßige Härtungswirkung in allen Schichten erzielt wird. Bei den nicht erfindungsgemäßen Härtungsmitteln V-1 und V-3 tritt beim Mischen des Netzmittels (anionisch) mit dem Härtungsmittel eine Entmischung auf, und ein Teil des Härtungsmittels wird in der Lösung ausgefällt. Dadurch wird eine inhomogene Gesamthärtung

erhalten, da das an sich gut wasserlösliche Härtungsmittel durch die Ausfällung mit dem anionischen Netzmittel festgelegt wird. Eine Härtung durch Überschichtung ist nicht mehr möglich und es tritt Runzelkorn auf.

## Patentansprüche

1. Lichtempfindliches fotografisches Aufzeichnungsmaterial mit mindestens einer gehärteten gelatinehaltigen Schicht, dadurch gekennzeichnet, daß zum Härten eine wasserlösliche Sulfatverbindung der allgemeinen Formel

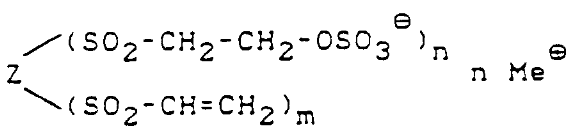

enthalten, worin

| | |
|---|---|
| Me | ein Metallkation, |
| n | eine ganze Zahl > 0, |
| m | eine ganze Zahl ≧ 0 und |
| m + n | 2 und |
| Z | eine der folgenden Formeln |

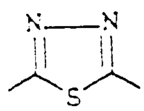
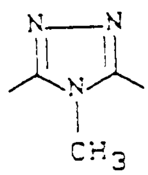
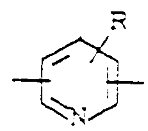

wobei R für $C_1$-$C_4$-Alkyl, Phenyl oder $C_1$-$C_4$-Alkoxy steht, bedeuten.

2. Lichtempfindliches fotografisches Aufzeichnungsmaterial nach Anspruch 1, bei dem 0,01 bis 15 Gew.-% Härtungsmittel, bezogen auf Trockengewicht der Gelatine, eingesetzt wurde und wobei anionische Netzmittel verwendet wurden.

3. Lichtempfindliches fotografisches Aufzeichnungsmaterial nach Anspruch 1, bei dem 0,1 bis 5 Gew.-% Härtungsmittel, bezogen auf Trockengewicht der Gelatine, eingesetzt wurde.

## Claims

1. A photosensitive photographic recording material comprising at least one hardened gelatine-containing layer, characterized in that a water-soluble sulfate compound corresponding to the general formula

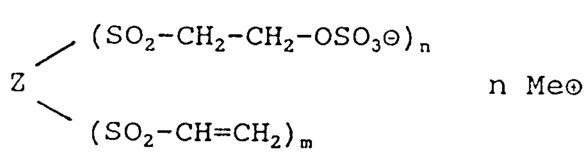

in which

| | |
|---|---|
| Me | is a metal cation, |
| n | is an integer of > 0, |
| m | is an integer of ≧ 0 and |
| m + n | = 2 and |
| Z | corresponds to one of the following formula |

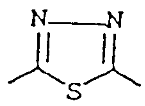

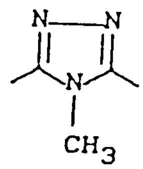

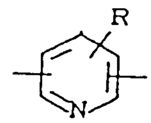

in which R represents $C_{1-4}$ alkyl, phenyl or $C_{1-4}$ alkoxy, is present for hardening.

2. A photosensitive photographic recording material as claimed in claim 1, in which 0.01 to 15% by weight hardener, based on the dry weight of the gelatine, was used and anionic wetting agents were used.

3. A photosensitive photographic recording material as claimed in claim 1, in which 0.1 to 5% by weight hardener, based on the dry weight of the gelatine, was used.

## Revendications

1. Support d'enregistrement photographique photosensible comportant au moins une couche durcie contenant de la gélatine, caractérisé par la présence, pour le durcissement, d'un composé du type d'un sulfate hydrosoluble de formule générale

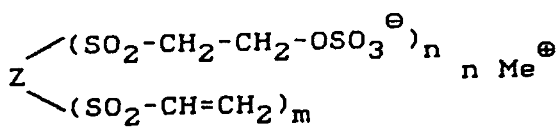

dans laquelle

| | |
|---|---|
| Me | est un cation métallique, |
| n | est un nombre entier supérieur à 0, |
| m | est un nombre entier supérieur ou égal à 0 et |
| m + n | est égal à 2 et |
| Z | répond à l'une des formules suivantes |

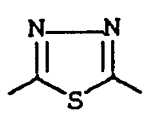

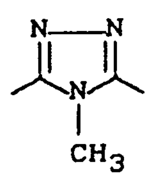

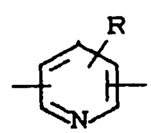

où R est un groupe alkyle en $C_1$ à $C_4$, phényle ou alkoxy en $C_1$ à $C_4$.

2. Support d'enregistrement photographique photosensible suivant la revendication 1, dans lequel une proportion de 0,01 à 15 % en poids d'agent durcissant, par rapport au poids sec de gélatine, a été incorporée et des agents mouillants anioniques ont été utilisés.

3. Support d'enregistrement photographique photosensible suivant la revendication 1, dans lequel a été incorporée une proportion de 0,1 à 5 % en poids d'agent durcissant, par rapport au poids sec de gélatine.